# FASCICULE DE BREVET EUROPEEN

(11) **EP 3 113 675 B1**
(45) Date de publication et mention de la délivrance du brevet: **04.12.2019**
(21) Numéro de dépôt: 15714566.5
(22) Date de dépôt: 04.03.2015
(51) Int. Cl.: G16H 10/60, G01N 35/00, H04L 29/08

(54) **PROCÉDÉ DE TRANSMISSION DE DONNÉES D'UNE ANALYSE DÉLOCALISÉE**
VERFAHREN ZUR ÜBERTRAGUNG VON DATEN EINER DELOKALISIERTEN ANALYSE
TRANSMISSION PROCESS OF DATA OF A DELOCALIZED ANALYSIS

(30) Priorité: 04.03.2014 FR 1451760
(43) Date de publication de la demande: 11.01.2017
(73) Titulaire: Podvin, Jean-Marie, 59310 Beuvry La Foret (FR)
(72) Inventeur: Podvin, Jean-Marie, 59310 Beuvry La Foret (FR)
(74) Mandataire: Balesta, Pierre
(86) Numéro de dépôt international: PCT/FR2015/050528
(87) Numéro de publication internationale: WO 2015/132528

(56) Documents cités:
- US-A1- 2005 228 245
- US-A1- 2007 273 504
- US-A1- 2008 070 599
- US-A1- 2012 182 939

## Description

### Arrière-plan de l'invention

La présente invention concerne le domaine général des analyses délocalisées, c'est-à-dire aux analyses réalisées en-dehors d'un laboratoire d'analyses. Plus particulièrement, la présente invention concerne le domaine des analyses biologiques, biochimiques ou bien encore médicales, délocalisées.

Il existe actuellement un besoin de pouvoir réaliser de manière fiable des analyses en situation délocalisée, afin de permettre une prise de décision rapide et moins couteuse. De telles analyses peuvent devoir être effectuées sur le lieu de prélèvement, par exemple à domicile ou dans la chambre d'hôpital dans le cas d'une analyse faite sur un patient.

Toutefois, de telles analyses, pour être validées, nécessitent le contrôle d'un biologiste ou biochimiste qui doit être en mesure de pouvoir vérifier le protocole qui a été mis en œuvre en situation délocalisée.

Il existe des appareils d'analyse biologique aptes à mesurer ou à évaluer une variable biologique en situation délocalisée. De tels appareils sont généralement installés en dehors d'un laboratoire et bien souvent en service ambulatoire voire chez le patient. Ce sont généralement des appareils portables, rapides et aisés d'utilisation. Il est notamment connu de réaliser un contrôle du fonctionnement de l'appareil ambulatoire afin de s'assurer de son bon fonctionnement.

Cependant, les analyses délocalisées présentent des sources d'erreur multiples dues à la réalisation en-dehors d'un laboratoire. Ainsi, même si un appareil ambulatoire vérifié est utilisé, les sources d'erreurs en amont, en aval ou même pendant l'utilisation de l'appareil sont encore possibles, modifiant ainsi le résultat de l'analyse ou de son interprétation.

### Objet et résumé de l'invention

La présente invention vise à résoudre les différents problèmes techniques énoncés précédemment. En particulier, la présente invention vise à proposer un procédé d'analyse délocalisée permettant un contrôle similaire à celui réalisé en laboratoire, c'est-à-dire un procédé permettant d'obtenir la même fiabilité qu'une analyse en laboratoire.

Ainsi, selon un aspect, il est proposé un procédé de transmission de données d'une analyse biologique ou biochimique, par exemple délocalisée, l'analyse comportant une phase pré-analytique et une phase analytique avec la mise en contact de l'échantillon avec un réactif et la révélation du résultat, ledit procédé comprenant:
- une étape d'acquisition de la mise en œuvre de la phase pré-analytique pour obtenir un enregistrement, de préférence vidéo ou audio,
- une étape d'acquisition du résultat obtenu lors de la mise en œuvre de la phase analytique, et
- une étape de transmission pour transmettre l'enregistrement et le résultat, de manière dématérialisée.

Ainsi, l'association du résultat de l'analyse avec la phase pré-analytique permet, lors du contrôle de l'analyse par un biologiste par exemple, de vérifier les différentes étapes du protocole mis en œuvre pour l'analyse, et donc de certifier sa mise en œuvre correcte. Le procédé selon l'invention permet donc de garantir la fiabilité du résultat obtenu en associant à celui-ci les étapes précédentes de mise en œuvre de l'analyse : on obtient donc une traçabilité de l'analyse effectuée.

Préférentiellement, le procédé comprend également une étape d'acquisition de données de géolocalisation, et l'étape de transmission comporte également la transmission des données de géolocalisation. Les données de géolocalisation permettent de connaître le lieu de réalisation de l'analyse, et ainsi de connaître certaines informations concernant la mise en œuvre de l'analyse. Par exemple, lorsque l'analyse est réalisée dans un véhicule d'assistance tel une ambulance, il est possible de savoir si le véhicule est à l'arrêt ou en mouvement pendant l'analyse, ce qui peut éventuellement modifier le résultat de celle-ci.

Préférentiellement, l'étape d'acquisition des données de géolocalisation s'effectue à plusieurs reprises durant l'analyse, par exemple par acquisition multiple de données de géolocalisation par satellite (GPS) et/ou par acquisition de données de déplacements obtenues par exemple par une centrale inertielle. Ainsi, le déplacement du lieu de réalisation de l'analyse peut être effectué par mesures successives de géolocalisation, ou par l'utilisation d'une centrale inertielle permettant de connaître les différents déplacements effectués.

Préférentiellement, le procédé comprend également une étape d'acquisition de données locales, par exemple la température, la pression, l'humidité de l'air, l'altitude, les conditions de stockage ou le conditionnement des réactifs avant la phase analytique, et l'étape de transmission comporte également la transmission des données locales. Les données locales permettent de connaître les conditions de mise en œuvre de la phase pré-analytique et de la phase analytique de l'analyse. Elles peuvent donc contribuer à l'interprétation du résultat de l'analyse, et peuvent donc être nécessaires pour la traçabilité.

Préférentiellement, le procédé comprend également une étape d'acquisition de l'heure et/ou de la date, et l'étape de transmission comporte également la transmission de la date et/ou de l'heure.

Préférentiellement, le procédé comprend également une étape d'acquisition d'éléments d'identification de l'opérateur effectuant l'analyse et/ou d'éléments d'identification des dispositifs utilisés pour la mise en œuvre de l'analyse ou pour la mise en œuvre de la transmission de données, et l'étape de transmission comporte également la transmission des éléments d'identification de l'opérateur et/ou des dispositifs utilisés. Les informations relatives à l'opérateur et/ou aux dispositifs utilisés pour la mise en œuvre de l'analyse, ont pour but de respecter des normes de qualité et de suivi des analyses effectuées.

Préférentiellement, la phase pré-analytique comporte la préparation d'un échantillon et éventuellement son conditionnement. Il s'agit des différentes étapes mises en œuvre avant la phase analytique et dont la mise en œuvre peut influer sur le résultat de l'analyse.

Préférentiellement, la phase analytique comporte la mise en contact de l'échantillon avec un réactif, la révélation du résultat, et éventuellement la préparation dudit réactif.

Préférentiellement, le procédé comprend également une étape d'acquisition d'éléments d'identification du sujet de l'analyse, et l'étape de transmission comporte également la transmission des éléments d'identification du sujet de l'analyse. Les éléments d'identification du sujet peuvent également comprendre des éléments connexes relatifs au sujet, comme par exemple les allergies ou maladies pour un patient.

Préférentiellement, le procédé comprend également, avant l'étape de transmission des données, une étape d'association des données et des éléments d'identification du sujet de l'analyse. L'association des éléments d'identification et des éléments relatifs à l'analyse permet de garantir l'origine de l'analyse, et éviter les permutations entre résultats d'analyse.

Préférentiellement, l'étape d'acquisition de la mise en œuvre de la phase pré-analytique et l'étape d'acquisition du résultat obtenu lors de la mise en œuvre de la phase analytique sont réalisées par enregistrement d'au moins une vidéo ou dans lequel l'étape d'acquisition du résultat obtenu lors de la mise en œuvre de la phase analytique est réalisée par l'enregistrement d'au moins une photographie. L'utilisation d'une vidéo et d'une photographie facilite l'acquisition des données pré-analytiques et analytiques, et permet de gagner du temps. Par ailleurs, le biologiste ou biochimiste en charge du contrôle de l'analyse sera en mesure de vérifier ou de repérer sur la vidéo des éléments auxquels l'opérateur n'aura pas prêté attention.

Selon un autre aspect, l'invention concerne également un procédé de réalisation d'une analyse biologique ou biochimique dans lequel on met en œuvre une étape pré-analytique, une étape analytique et un procédé de transmission des données de l'analyse décrit précédemment.

Selon un autre aspect, l'invention concerne également une utilisation d'un dispositif d'enregistrement, de préférence vidéo, et éventuellement photos, et de transmission de données dématérialisée, pour mettre en œuvre le procédé de transmission de données décrit précédemment.

### Brève description des dessins

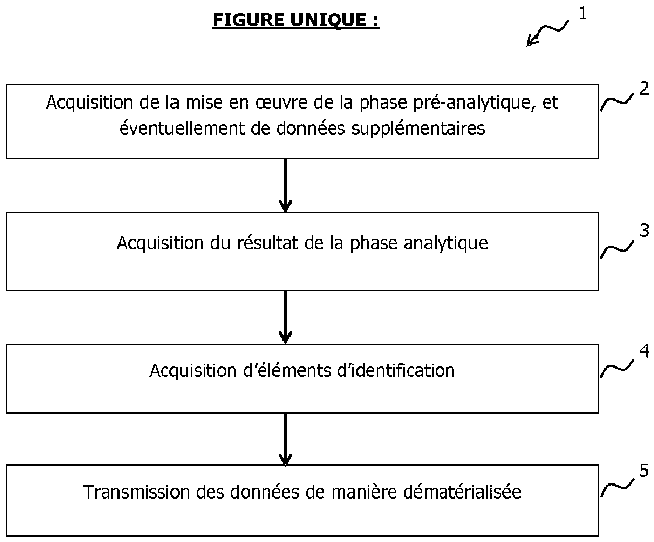

L'invention et ses avantages seront mieux compris à la lecture de la description détaillée d'un mode de réalisation particulier, pris à titre d'exemple nullement limitatif et illustré par le dessin annexé sur lequel sont représentées les différentes étapes successives d'un procédé selon l'invention.

### Description détaillée de l'invention

La figure annexée illustre un exemple de mise en œuvre d'un procédé 1 de transmission de données d'une analyse biologique ou biochimique selon l'invention.

Le procédé comprend ainsi une première étape 2 d'acquisition de la mise en œuvre de la phase pré-analytique de l'analyse biologique ou biochimique. La phase pré-analytique peut comprendre notamment le prélèvement d'un échantillon à analyser, et sa préparation en vue de l'analyse biologique ou biochimique à réaliser.

Par exemple, la phase pré-analytique peut comprendre le prélèvement d'un échantillon sur une plante en vue de dépister la présence d'une maladie ou l'absence de composés chimiques nocifs ou indésirables.

L'acquisition de la mise en œuvre de la phase pré-analytique peut comprendre un enregistrement vidéo ou audio. On peut ainsi enregistrer les différentes actions mises en œuvre lors du prélèvement et/ou préparation de l'échantillon, qui pourront ensuite être analysées et validées par une personne habilitée à valider l'analyse, par exemple un laborantin.

Plus précisément, l'acquisition de la mise en œuvre de la phase pré-analytique peut permettre une meilleure interprétation de la phase analytique, et sa validation, même si la personne ayant réalisée la phase pré-analytique n'est pas la même personne que celle interprétant et validant la phase analytique. Il est alors possible de valider une analyse biologique ou biochimique réalisée par une personne non-habilitée à valider l'analyse.

La première étape 2 peut également comprendre l'acquisition d'informations supplémentaires, telles que la température, la pression, la géolocalisation, l'humidité de l'air, l'altitude, le conditionnement ou les conditions de stockage du ou des réactifs avant utilisation, etc. Il s'agit ici de conditions de réalisation de la phase pré-analytique ou analytique susceptibles d'altérer ou de modifier le résultat de la phase analytique. Ces informations peuvent être alors considérées en même temps que la vidéo d'acquisition de la phase pré-analytique, afin de permettre une interprétation et une analyse plus complète et plus précise des résultats de la phase analytique.

Le procédé 1 comprend ensuite une deuxième étape 3 d'acquisition du résultat de la phase analytique. La phase analytique peut comprendre notamment la mise en contact de l'échantillon à analyser et du ou des réactifs, et la préparation du ou des réactifs en vue de l'analyse biologique ou biochimique à réaliser.

Par exemple, la phase analytique peut comprendre le dépôt de l'échantillon dans le réactif, sa mise en contact avec une électrode ou avec une plaque imbibée de réactif. La phase analytique peut également comprendre l'analyse de l'échantillon par une machine, par exemple par un dispositif d'analyse du taux d'alcoolémie contenu dans l'air expiré par une personne.

L'analyse réalisée peut ainsi être la détermination de la glycémie, avant injection d'insuline, ou bien encore la détermination de la concentration en hémoglobine ou hématocrite avant un protocole d'hémodilution ou de transfusion.

L'acquisition de la phase analytique peut comprendre un enregistrement vidéo de la révélation du résultat, ou bien une photographie du résultat obtenu à l'issue de la phase analytique, ou bien encore l'enregistrement du résultat fourni par une machine. C'est notamment ce résultat, sous forme vidéo ou sous forme photographique ou sous une autre forme, par exemple électronique, qui peut faire l'objet, en combinaison avec la phase pré-analytique, d'une interprétation et d'une validation par une personne habilitée, par exemple un biologiste ou un laborantin.

Le procédé 1 peut également comprendre une troisième étape 4 d'acquisition d'éléments d'identification. De tels éléments d'identifications permettent d'associer les résultats de la phase analytique à un individu ou à un échantillon identifié. Les éléments d'identification peuvent ainsi être l'état civil de la personne faisant l'objet de l'analyse, ou bien encore de la personne effectuant l'analyse.

Il peut s'agir également de la date et de l'heure de l'analyse ou bien encore du lieu de réalisation de l'analyse, permettant de remonter dans un second temps à l'objet de l'analyse.

Il peut également s'agir d'un code pour lequel seul le donneur d'ordre de l'analyse connaît la correspondance avec l'échantillon analysé. Il est alors possible de garder secrets certains éléments de l'analyse, tels que la provenance, sans compromettre le résultat de l'analyse.

Les éléments d'identifications peuvent également comprendre des éléments relatifs à l'appareillage utilisé pour réaliser la phase analytique et/ou pour réaliser la transmission des données. Il est ainsi également possible de connaître également les spécifications de l'appareillage utilisé, et ses limites techniques pouvant être prises en compte pour l'interprétation de l'analyse.

Alternativement, les éléments d'identification peuvent être enregistrés lors de l'étape d'acquisition de la phase pré-analytique, par exemple par enregistrement vidéo de moyens d'identification tels qu'une carte d'identité ou un code-barres correspondant à l'échantillon analysé.

Alternativement, les éléments d'identifications peuvent également comprendre des informations liées à l'individu, par exemple les allergies éventuelles, les examens pouvant être pratiqués sur lui et toutes autres informations susceptibles d'être prises en compte pour l'interprétation des résultats ou pour la suite à donner à l'analyse.

On comprend ainsi que l'étape d'acquisition d'éléments d'identification est facultative, et peut être mise en œuvre à n'importe quel moment avant l'étape de transmission des informations, par exemple avant ou pendant l'étape d'acquisition de la mise en œuvre de la phase pré-analytique.

Enfin, dans une dernière étape 5, le procédé 1 comprend une étape de transmission des données acquises lors des étapes 2 à 4, de manière dématérialisée. On entend par dématérialisée toute méthode permettant de transmettre des informations sans support physique tel que CD, DVD ou papier. Il peut s'agir par exemple d'une transmission sans fil, via les réseaux de téléphonie mobile par exemple, ou via d'autres protocoles de communication sans fil tels que les transmissions WIFI ou BLUETOOTH. Il peut également s'agir d'une transmission filaire via les réseaux téléphoniques ou via internet.

Le but de l'étape de transmission de manière dématérialisée est de permettre la mise en œuvre de l'analyse en situation délocalisée, à distance de la personne habilitée à valider le résultat de l'analyse obtenu. La transmission de manière dématérialisée permet notamment de transmettre rapidement les éléments nécessaires à la validation de l'analyse, sans altération extérieure.

Selon un mode de réalisation, les différents éléments acquis lors des étapes 2 à 4 sont associés avant l'étape 5 de transmission des données. Par exemple, les éléments d'identification peuvent être associés de manière définitive, par exemple par tatouage de l'enregistrement de la phase pré-analytique ou du résultat de la phase analytique avec le nom du patient analysé. L'association des éléments d'identification avec les éléments d'analyse permet de s'assurer que les résultats obtenus et validés ne seront pas associés, par erreur ou par manipulation, à une autre analyse. Il est alors possible de certifier le résultat obtenu, par rapport à une analyse déterminée.

Alternativement, le procédé peut également comprendre, avant l'étape de transmission, une étape de cryptage de toute ou partie des informations transmises, afin de garantir d'une part leur confidentialité et d'autre part leur origine sans altération ou modification ultérieure avant validation.

L'invention concerne également un procédé de réalisation d'une analyse biologique ou biochimique dans lequel on met en œuvre une étape pré-analytique, une étape analytique et le procédé 1 de transmission des données de l'analyse décrit précédemment. En particulier, l'étape de réalisation de la phase pré-analytique et l'étape d'acquisition de la phase pré-analytique sont mises en œuvre simultanément.

Pour la mise en œuvre du procédé selon l'invention, il est possible d'utiliser tout dispositif d'acquisition et de transmission. Ainsi, le dispositif utilisé peut être un dispositif connecté, par exemple un téléphone équipé d'un appareil photo et éventuellement d'une puce de géolocalisation, capable de transmettre des informations telles que des vidéos et/ou des photographies par les réseaux de téléphonie mobile.

Ainsi, grâce au procédé selon l'invention, il devient possible de réaliser des analyses, à la fois médicales, biologiques ou biochimiques, en situation délocalisée, tout en gardant une validation du résultat obtenu par une personne habilitée grâce à une traçabilité du protocole utilisé pour réaliser l'analyse. Par ailleurs, en utilisant un enregistrement sous format vidéo et en associant les différentes informations entre elles, on garantit l'absence de modifications des données avant validation, et donc on certifie le résultat obtenu.

## Revendications

1. Procédé (1) de transmission de données d'une analyse biologique ou biochimique, par exemple délocalisée, l'analyse comportant une phase pré-analytique et une phase analytique avec la mise en contact de l'échantillon avec un réactif et la révélation du résultat, ledit procédé comprenant :
- une étape (2) d'acquisition de la mise en œuvre de la phase pré-analytique pour obtenir un enregistrement vidéo, puis
- une étape (3) d'acquisition du résultat obtenu lors de la mise en œuvre de la phase analytique, et
- une étape (5) de transmission pour transmettre l'enregistrement et le résultat, de manière dématérialisée.

2. Procédé (1) selon la revendication 1 comprenant également une étape d'acquisition de données de géolocalisation, et dans lequel l'étape (5) de transmission comporte également la transmission des données de géolocalisation.

3. Procédé (1) selon la revendication 2 dans lequel l'étape d'acquisition des données de géolocalisation s'effectue à plusieurs reprises durant l'analyse, par exemple par acquisition multiple de données de géolocalisation par satellite et/ou par acquisition de données de déplacements obtenues par exemple par une centrale inertielle.

4. Procédé (1) selon l'une quelconque des revendications 1 à 3 comprenant également une étape d'acquisition de données locales, par exemple la température, la pression, l'humidité de l'air, l'altitude, et dans lequel l'étape (5) de transmission comporte également la transmission des données locales.

5. Procédé (1) selon l'une quelconque des revendications 1 à 4 comprenant également une étape d'acquisition de l'heure et/ou de la date, et dans lequel l'étape (5) de transmission comporte également la transmission de la date et/ou de l'heure.

6. Procédé (1) selon l'une quelconque des revendications **1** à **5** comprenant également une étape d'acquisition d'éléments d'identification de l'opérateur effectuant l'analyse et/ou d'éléments d'identification des dispositifs utilisés pour la mise en œuvre de l'analyse ou pour la mise en œuvre de la transmission de données, et dans lequel l'étape (5) de transmission comporte également la transmission des éléments d'identification de l'opérateur et/ou des dispositifs utilisés.

7. Procédé (1) selon l'une quelconque des revendications précédentes dans lequel la phase pré-analytique comporte la préparation et/ou le conditionnement de l'échantillon prélevé.

8. Procédé (1) selon la revendication **7** dans lequel la phase analytique comporte également la préparation dudit réactif.

9. Procédé (1) selon l'une quelconque des revendications **1** à **8** comprenant également une étape (4) d'acquisition d'éléments d'identification du sujet de l'analyse, et dans lequel l'étape (5) de transmission comporte également la transmission des éléments d'identification du sujet de l'analyse.

10. Procédé (1) selon la revendication **9** comprenant également, avant l'étape (5) de transmission des données, une étape d'association des données et des éléments d'identification du sujet de l'analyse.

11. Procédé (1) selon l'une quelconque des revendications précédentes dans lequel l'étape (2) d'acquisition de la mise en œuvre de la phase pré-analytique et l'étape (3) d'acquisition du résultat obtenu lors de la mise en œuvre de la phase analytique sont réalisées par enregistrement d'au moins une vidéo ou dans lequel l'étape (3) d'acquisition du résultat obtenu lors de la mise en œuvre de la phase analytique est réalisée par

12. Procédé de réalisation d'une analyse biologique ou biochimique dans lequel on met en œuvre une étape pré-analytique, une étape analytique et un procédé (1) de transmission des données de l'analyse selon l'une quelconque des revendications précédentes.

13. Utilisation d'un dispositif d'enregistrement, de préférence vidéo et éventuellement photos, et de transmission de données dématérialisée, pour mettre en œuvre le procédé (1) de transmission de données selon l'une quelconque des revendications **1** à **11.**

## Patentansprüche

1. Verfahren (1) zur Übertragung von Daten einer, zum Beispiel delokalisierten, biologischen oder biochemischen Analyse, wobei die Analyse eine präanalytische Phase und eine analytische Phase mit dem Inkontaktbringen der Probe mit einem Reagens und der Entwicklung des Ergebnisses umfasst, wobei das Verfahren umfasst:
- einen Schritt (2) des Erfassens der Durchführung der präanalytischen Phase zum Erhalten einer Video-Aufzeichnung, dann
- einen Schritt (3) des Erfassens des bei der Durchführung der analytischen Phase erhaltenen Ergebnisses und
- einen Übertragungsschritt (5) zum Übertragen der Aufzeichnung und des Ergebnisses auf dematerialisierte Weise.

2. Verfahren (1) nach Anspruch 1, das auch einen Schritt des Erfassens von Geolokalisierungsdaten umfasst und wobei der Übertragungsschritt (5) auch die Übertragung der Geolokalisierungsdaten umfasst.

3. Verfahren (1) nach Anspruch 2, wobei der Schritt des Erfassens der Geolokalisierungsdaten während der Analyse mehrere Male durchgeführt wird, zum Beispiel durch mehrfaches Erfassen von Geolokalisierungsdaten per Satellit und/oder durch Erfassen von Daten von Verlegungen, die zum Beispiel durch ein Trägheitsnavigationssystem erhalten werden.

4. Verfahren (1) nach einem der Ansprüche 1 bis 3, das auch einen Schritt des Erfassens von lokalen Daten, zum Beispiel der Temperatur, des Drucks, der Luftfeuchtigkeit, der Höhe, umfasst und wobei der Übertragungsschritt (5) auch die Übertragung der lokalen Daten umfasst.

5. Verfahren (1) nach einem der Ansprüche 1 bis 4, das auch einen Schritt des Erfassens der Uhrzeit und/oder des Datums umfasst und wobei der Übertragungsschritt (5) auch die Übertragung des Datums und/oder der Uhrzeit umfasst.

6. Verfahren (1) nach einem der Ansprüche 1 bis 5, das auch einen Schritt des Erfassens von Elementen zur Identifikation des Bedieners, der die Analyse durchführt, und/oder von Elementen zur Identifikation der Vorrichtungen umfasst, die für die Durchführung der Analyse oder für die Durchführung der Übertragung der Daten verwendet werden, wobei der Übertragungsschritt (5) auch die Übertragung der Elemente zur Identifikation des Bedieners und/oder der verwendeten Vorrichtungen umfasst.

7. Verfahren (1) nach einem der vorhergehenden Ansprüche, wobei die präanalytische Phase die Vorbereitung und/oder die Behandlung der entnommenen Probe umfasst.

8. Verfahren (1) nach Anspruch 7, wobei die analytische Phase auch die Vorbereitung des Reagens umfasst.

9. Verfahren (1) nach einem der Ansprüche 1 bis 8, das auch einen Schritt (4) des Erfassens von Elementen zur Identifikation des Subjekts der Analyse umfasst und wobei der Übertragungsschritt (5) auch die Übertragung der Elemente zur Identifikation des Subjekts der Analyse umfasst.

10. Verfahren nach Anspruch 9, das vor dem Schritt (5) der Übertragung der Daten auch einen Schritt des Verknüpfens der Daten und der Elemente zur Identifikation des Subjekts der Analyse umfasst.

11. Verfahren (1) nach einem der vorhergehenden Ansprüche, wobei der Schritt (2) des Erfassens der Durchführung der präanalytischen Phase und der Schritt (3) des Erfassens des bei der Durchführung der analytischen Phase erhaltenen Ergebnisses durch Aufzeichnung von mindestens einem Video ausgeführt werden oder wobei der Schritt (3) des Erfassens des bei der Durchführung der analytischen Phase erhaltenen Ergebnisses durch die Aufzeichnung von mindestens einer Fotografie ausgeführt wird.

12. Verfahren zur Ausführung einer biologischen oder biochemischen Analyse, wobei ein präanalytischer Schritt, ein analytischer Schritt und ein Verfahren (1) zur Übertragung der Daten der Analyse nach einem der vorhergehenden Ansprüche durchgeführt werden.

13. Verwendung einer Vorrichtung zur Aufzeichnung, vorzugsweise von Video und gegebenenfalls Fotos, und zur dematerialisierten Übertragung von Daten zum Durchführen des Verfahrens (1) zur Übertragung von Daten nach einem der Ansprüche 1 bis 11.

## Claims

1. A method (1) for transmitting data of a biological or biochemical analysis, for example at the point-of-care, the analysis including a pre-analytical phase and an analytical phase with the contacting of the sample with a reagent and the development of the result, said method comprising:
- a step (2) for acquiring the implementation of the pre-analytical phase in order to obtain a video recording, then
- a step (3) for acquiring the obtained result during the implementation of the analytical phase, and
- a step (5) for transmission in order to transmit the recording and the result, intangibly.

2. The method (1) according to claim **1** also comprising a step for acquiring geolocalization data, and wherein the transmission step (5) also includes transmission of geolocalization data.

3. The method (1) according to claim **2** wherein the step for acquiring geolocalization data is carried out several times during the analysis, for example by multiple acquisition of geolocalization data by satellite and/or by acquiring displacement data for example obtained with an inertial unit.

4. The method (1) according to any of claims **1** to **3** also comprising a step for acquiring local data, for example temperature, pressure, humidity of the air, altitude and wherein the transmission step (5) also includes transmission of local data.

5. The method (1) according to any of claims **1** to **4** also comprising a step for acquiring the time and/or the date, and wherein the transmission step (5) also includes transmission of the date and/or of the time.

6. The method (1) according to any of claims **1** to **5** also comprising a step for acquiring identification elements of the operator conducting the analysis and/or identification elements of the devices used for implementation of the analysis or for implementation of the data transmission, and wherein the transmission step (5) also includes transmission of the identification elements of the operator and/or of the devices used.

7. The method (1) according to any of the preceding claims, wherein the pre-analytical phase includes the preparation and/or the conditioning of the sample.

8. The method (1) according to claim **7** wherein the analytical phase also includes the preparation of said reagent.

9. The method (1) according to any of claims **1** to **8** also comprising a step (4) for acquiring identification elements of the subject of the analysis, and wherein the transmission step (5) also includes transmission of the identification elements of the subject of the analysis.

10. The method (1) according to claim **9** also comprising, before the data transmission step (5), a step for associating the data and the identification elements of the subject of the analysis.

11. The method (1) according to any of the preceding claims wherein the step (2) for acquiring the implementation of the pre-analytical phase and the step (3) for acquiring the obtained result during the implementation of the analytical phase are carried out by recording at least one video or wherein the step (3) for acquiring the obtained result during the implementation of the analytical phase is carried out by recording at least one photograph.

12. A method for carrying out a biological or biochemical analysis wherein a pre-analytical step, an analytical step and an analysis data transmission method (1) according to any of the preceding claims are implemented.

13. The use of a recording device, preferably a video recording device and optionally photographs, and for transmitting data intangibly, for implementing the method (1) for transmitting data according to any of claims **1** to **11.**
